# EUROPEAN PATENT SPECIFICATION

(11) **EP 2 081 897 B1**
(45) Date of publication and mention of the grant of the patent: **15.09.2010**
(21) Application number: 07821299.0
(22) Date of filing: 15.10.2007
(51) Int. Cl.: C07D 209/66, A61K 31/4035, A61P 29/00

(54) **ISOINDOL DERIVATIVES AS EP4 RECEPTOR AGONISTS**
ISOINDOLDERIVATE ALS EP4-REZEPTORAGONISTEN
DÉRIVÉS D'ISO-INDOLE EN TANT QU'AGONISTES DES RÉCEPTEURS EP4

(30) Priority: 17.10.2006 GB 0620619
(43) Date of publication of application: 29.07.2009
(73) Proprietor: Glaxo Group Limited, Greenford Middlesex UB6 0NN (GB)
(72) Inventor: GIBLIN, Gerard, Martin, Paul, Harlow Essex CM19 5AW (GB); HEALY, Mark, Patrick, Harlow Essex CM19 5AW (GB)
(74) Representative: Knight, Lucie Viktoria
(86) International application number: PCT/EP2007/060932
(87) International publication number: WO 2008/046798

(56) References cited:
- EP-A- 1 202 730
- WO-A-02/064564
- WO-A-2007/088189
- WO-A-2007/088190

## Description

This invention relates to indole derivatives, to processes for their preparation, to pharmaceutical compositions containing them and to their use in medicine.

The compounds of the present invention are EP₄ receptor agonists.

A number of review articles describe the characterization and therapeutic relevance of the prostanoid receptors as well as the most commonly used selective agonists and antagonists: Eicosanoids; From Biotechnology to Therapeutic Applications, Folco, Samuelsson, Maclouf, and Velo eds, Plenum Press, New York, 1996, chap. 14, 137-154 and Journal of Lipid Mediators and Cell Signalling, 1996, 14, 83-87 and Prostanoid Receptors, Structure, Properties and Function, S Narumiya et al, Physiological Reviews 1999, 79(4), 1193-126.

The EP₄ receptor is a 7-transmembrane receptor and its natural ligand is the prostaglandin PGE₂. PGE₂ also has affinity for the other EP receptors (types EP₁, EP₂ and EP₃). The prostanoid EP₄ receptor falls into a group of receptors normally associated with elevation of intracellular cyclic adenosine monophosphate (cAMP) levels. The EP₄ receptor is associated with smooth muscle relaxation, intraocular pressure, pain (in particular inflammatory, neuropathic and visceral pain), inflammation, neuroprotection, lymphocyte differentiation, bone metabolic processes, allergic activities, promotion of sleep, renal regulation, gastric or enteric mucus secretion and duodenal bicarbonate secretion. The EP₄ receptor plays an important role in closure of the ductus arteriosus, vasodepression, inflammation and bone remodeling as reviewed by Narumiya in Prostaglandins & Other Lipid Mediators 2002, 68-69 557-73.

A number of publications have demonstrated that PGE₂ acting through the EP₄ receptor subtype, and EP₄ agonists alone, can regulate inflammatory cytokines after an inflammatory stimulus. Takayama et al in the Journal of Biological Chemistry 2002, 277(46), 44147-54 showed PGE₂ modulates inflammation during inflammatory diseases by suppressing macrophage derived chemokine production via the EP₄ receptor. In Bioorganic & Medicinal Chemistry 2002, 10(7), 2103-2110, Maruyama et al demonstrate the selective EP4 receptor agonist (ONO-AE1-437) suppresses LPS induced TNF-α in human whole blood whilst increasing the levels of IL-10. An article in Anesthesiology, 2002, 97,170-176 suggests that a selective EP₄ receptor agonist (ONO-AE1-329) effectively inhibited mechanical and thermal hyperalgesia and inflammatory reactions in acute and chronic monoarthritis.

Two independent articles from Sakuma et al in Journal of Bone and Mineral Research 2000, 15(2), 218-227 and Miyaura et al in Journal of Biological Chemistry 2000, 275(26), 19819-23, report impaired osteoclast formation in cells cultured from EP₄ receptor knock-out mice. Yoshida et al in Proceedings of the National Academy of Sciences of the United States of America 2002, 99(7), 4580-4585, by use of mice lacking each of the PGE₂ receptor EP subtypes, identified EP₄ as the receptor that mediates bone formation in response to PGE₂ administration. They also demonstrated a selective EP₄ receptor agonist (ONO-4819) consistently induces bone formation in wild type mice. Additionally, Terai et al in Bone 2005, 37(4), 555-562 have shown the presence of a selective EP₄ receptor agonist (ONO-4819) enhanced the bone-inducing capacity of rhBMP-2, a therapeutic cytokine that can induce bone formation.

Further research by Larsen *et al* shows the effects of PGE₂ on secretion in the second part of the human duodenum is mediated through the EP₄ receptor (Acta. Physiol: Scand. 2005, 185, 133-140). Also, it has been shown a selective EP₄ receptor agonist (ONO-AE1-329) can protect against colitis in rats (Nitta et al in Scandinavian Journal of Immunology 2002, 56(1), 66-75).

Doré et al in The European Journal of Neuroscience 2005, 22(9), 2199-206 have shown that PGE₂ can protect neurons against amyloid beta peptide toxicity by acting on EP₂ and EP₄ receptors. Furthermore Doré has demonstrated in Brain Research 2005, 1066(1-2), 71-77 that an EP₄ receptor agonist (ONO-AE1-329) protects against neurotoxicity in an acute model of excitotoxicity in the brain. Woodward et al in Journal of Lipid Mediators 1993, 6(1-3), 545-53 found intraocular pressure could be lowered using selective prostanoid agonists. Two papers in *Investigative Ophthalmology & Visual Science* have shown the prostanoid EP₄ receptor is expressed in human lens epithelial cells (Mukhopadhyay et al 1999, 40(1), 105-12), and suggest a physiological role for the prostanoid EP₄ receptor in modulation of flow in the trabecular framework of the eye (Hoyng et al 1999, 40(11), 2622-6).

Compounds exhibiting EP₄ receptor binding activity have been described in, for example, WO98/55468, WO00/18744, WO00/03980, WO00/15608, WO0016760, WO00/21532, EP0855389, EP0985663, WO02/50031, WO02/50032, WO02/50033, WO02/064564, WO03/103604, WO03/077910, WO03/086371, WO04/037813, WO04/067524, WO04/085430, US04/142969, WO05/021508, WO05/105733, WO05/105732, WO05/080367, WO05/037812, WO05/116010 and WO06/122403.

Derivatives of indoprofen such as [4-(1-oxo-1,3-dihydro-2H-benzo[f]isoindol-2-yl)phenyl]-2-propionic acid, sodium salt have been described by Rufer et. al. in Eur. J. Med. Chem. - Chimica Therapeutica, 1978, 13, 193.

Compounds of the present invention have been shown to exhibit advantageous *in vivo* and *in vitro* activities when tested in the biological assays described herein.

The present invention provides a compound selected from the group consisting of:
{4-[4,9-Bis(ethyloxy)-1,3-dioxo-1,3-dihydro-2*H*-benzo[*f*]isoindol-2-yl]-3-chlorophenyl}acetic acid;
{3-Chloro-4-[1,3-dioxo-4,9-bis(propyloxy)-1,3-dihydro-2*H*-benzo[*f*]isoindol-2-yl]phenyl}acetic acid;
(4-{4,9-Bis(1-methylethoxy)-1,3-dioxo-1,3-dihydro-2*H*-benzo[*f*]isoindol-2-yl}-3-chlorophenyl)acetic acid;
{4-[4,9-Bis(ethyloxy)-1,3-dioxo-1,3-dihydro-2*H*-benzo[*f*]isoindol-2-yl]-3-bromophenyl}acetic acid;
{3-Bromo-4-[1,3-dioxo-4,9-bis(propyloxy)-1,3-dihydro-2*H*-benzo[*f*]isoindol-2-yl]phenyl}acetic acid;
(4-{4,9-Bis(1-methylethoxy)-1,3-dioxo-1,3-dihydro-2H-benzo[*f*]isoindol-2-yl}-3-bromophenyl)acetic acid;
(4-{4,9-Bis(1-methylethoxy)-1-oxo-1,3-dihydro-2*H*-benzo[*f*]isoindol-2-yl}-3-chlorophenyl)acetic acid;
{3-Chloro-4-[1-oxo-4,9-bis(propyloxy)-1,3-dihydro-2*H*-benzo[*f*]isoindol-2-yl]phenyl}acetic acid;
{3-Bromo-4-[1-oxo-4,9-bis(propyloxy)-1,3-dihydro-2*H*-benzo[*t*]isoindol-2-yl]phenyl}acetic acid;
(4-{4,9-Bis(1-methylethoxy)-1-oxo-1,3-dihydro-2*H*-benzo[*f*]isoindol-2-yl}-3-bromophenyl)acetic acid; and salts thereof (hereafter 'the compounds of the invention').

It will be appreciated that, for pharmaceutical use, the salts referred to above will be the pharmaceutically acceptable salts, but other salts may find use, for example in the preparation of compounds of the invention and the pharmaceutically acceptable salts thereof.

Pharmaceutically acceptable salts include those described by Berge, Bighley and Monkhouse, J. Pharm. Sci., 1977, 66, 1-19. The term "pharmaceutically acceptable salts" refers to salts prepared from pharmaceutically acceptable bases including inorganic bases and organic bases. Salts derived from inorganic bases include aluminum, ammonium, calcium, copper, ferric, ferrous, lithium, magnesium, manganic salts, manganous, potassium, sodium, zinc, and the like. Salts derived from pharmaceutically acceptable organic bases include salts of primary, secondary, and tertiary amines; substituted amines including naturally occurring substituted amines; and cyclic amines. Particular pharmaceutically acceptable organic bases include arginine, betaine, caffeine, choline, N,N'-dibenzylethylenediamine, diethylamine, 2-diethylaminoethanol, 2-dimethylaminoethanol, ethanolamine, ethylenediamine, N-ethyl-morpholine, N-ethylpiperidine, glucamine, glucosamine, histidine, hydrabamine, isopropylamine, lysine, methylglucamine, morpholine, piperazine, piperidine, procaine, purines, theobromine, triethylamine, trimethylamine, tripropyl amine, tris(hydroxymethyl)aminomethane, and the like. Salts may also be formed from basic ion exchange resins, for example polyamine resins.

It will be appreciated that the compounds of the invention may be produced in vivo by metabolism of a suitable prodrug. Such prodrugs may be for example physiologically acceptable metabolically labile esters of the compounds of the invention. These may be formed by esterification of the carboxylic acid group in the parent compound of the invention with, where appropriate, prior protection of any other reactive groups present in the molecule followed by deprotection if required. Examples of such metabolically labile esters include C₁₋₄ alkyl esters e.g. methyl ethyl or t-butyl esters esters, C₃₋₆ alkenyl esters e.g. allyl substituted or unsubstituted aminoalkyl esters (e.g. aminoethyl, 2-(N,N- diethylamino) ethyl, or 2-(4-morpholino)ethyl esters or acyloxyalkyl esters such as, acyloxymethyl or 1-acyloxyethyl e.g. pivaloyloxymethyl, 1-pivaloyloxyethyl, acetoxymethyl, 1-acetoxyethyl,1-(1-methoxy-1-methyl)ethylcarbonyloxyethyl, 1-benzoyloxyethyl, isopropoxycarbonyloxymethyl, 1-isopropoxycarbonyloxyethyl, cyclohexylcarbonyloxymethyl, 1-cyclohexylcarbonyloxyethyl ester, cyclohexyloxycarbonyloxymethyl, 1-cyclohexyloxycarbonyloxyethyl, 1-(4-tetrahydropyranyloxy)carbonyloxyethyl or 1-(4-tetrahydropyranyl)carbonyloxyethyl.

It is to be understood that the present invention encompasses all isomers of the compounds of the invention and their salts, including all geometric, tautomeric and optical forms, and mixtures thereof (e.g. racemic mixtures).

Since the compounds of the invention are intended for use in pharmaceutical compositions, it will be understood that they are each provided in substantially pure form, for example at least 50% pure, more suitably at least 75% pure and preferably at least 95% pure (% are on a wt/wt basis). Impure preparations of the compounds of the invention may be used for preparing the more pure forms used in the pharmaceutical compositions. Although the purity of intermediate compounds of the present invention is less critical, it will be readily understood that the substantially pure form is preferred as for the compounds of the invention. Preferably, whenever possible, the compounds of the present invention are obtained in crystalline form.

When some of the compounds of this invention are allowed to crystallise or are recrystallised from organic solvents, solvent of crystallisation may be present in the crystalline product. This invention includes within its scope such solvates. Similarly; some of the compounds of this invention may be crystallised or recrystallised from solvents containing water. In such cases water of hydration may be formed. This invention includes within its scope stoichiometric hydrates as well as compounds containing variable amounts of water that may be produced by processes such as lyophilisation. In addition, different crystallisation conditions may lead to the formation of different polymorphic forms of crystalline products. This invention includes within its scope all polymorphic forms of the compounds of the invention.

The present invention also includes within its scope all isotopically-labelled compounds of the invention. Such compounds are identical to those recited in the list of the compounds of the invention except that one or more atoms therein are replaced by an atom having an atomic mass or mass number different from the atomic mass or mass number usually found in nature. Examples of isotopes that can be incorporated into compounds of the invention and salts thereof include isotopes of hydrogen, carbon, nitrogen, oxygen and chlorine, such as 2H, 3H, 11C, 13C, 14C, 15N, 17O, 18O and 36Cl.

Isotopically-labelled compounds of the invention, for example those into which radioactive isotopes such as 3H, 14C are incorporated, are useful in drug and/or substrate tissue distribution assays. Tritiated, i.e., 3H, and carbon-14, i.e., 14C, isotopes are particularly preferred for their ease of preparation and detectability. The 11C isotope is particularly useful in PET (positron emission tomography) and is useful in brain imaging. Further substitution with heavier isotopes such as deuterium, i.e., 2H, can afford certain therapeutic advantages resulting from greater metabolic stability, for example increased *in vivo* half-life or reduced dosage requirements and, hence, may be preferred in some circumstances. Isotopically labelled compounds of the invention may be prepared by carrying out the synthetic procedures disclosed in the Schemes and/or in the Examples below, by substituting a readily available isotopically labelled reagent for a non-isotopically labelled reagent.

The compounds of the invention are EP₄ receptor agonists and may therefore be useful in treating EP₄ receptor mediated diseases.

In particular the compounds of the invention may be useful in the treatment of pain, for example, chronic articular pain (e.g. rheumatoid arthritis, osteoarthritis, rheumatoid spondylitis, gouty arthritis and juvenile arthritis) including the property of disease modification and joint structure preservation; musculoskeletal pain; lower back and neck pain; sprains and strains; neuropathic pain; sympathetically maintained pain; myositis; pain associated with cancer and fibromyalgia; pain associated with migraine; pain associated with influenza or other viral infections, such as the common cold; rheumatic fever; pain associated with functional bowel disorders such as non-ulcer dyspepsia, non-cardiac chest pain and irritable bowel syndrome; pain associated with myocardial ischemia; post operative pain; headache; toothache; and dysmenorrhea.

The compounds of the invention may be particularly useful in the treatment of neuropathic pain and symptoms associated therewith. Neuropathic pain syndromes include: diabetic neuropathy; sciatica; non-specific lower back pain; multiple sclerosis pain; fibromyalgia; HIV-related neuropathy; post-herpetic neuralgia; trigeminal neuralgia; and pain resulting from physical trauma, amputation, cancer, toxins or chronic inflammatory conditions. Symptoms of neuropathic pain include spontaneous shooting and lancinating pain, or ongoing, burning pain. In addition, there is included pain associated with normally non-painful sensations such as "pins and needles" (paraesthesias and dysesthesias), increased sensitivity to touch (hyperesthesia), painful sensation following innocuous stimulation (dynamic, static or thermal allodynia), increased sensitivity to noxious stimuli (thermal, cold, mechanical hyperalgesia), continuing pain sensation after removal of the stimulation (hyperpathia) or an absence of or deficit in selective sensory pathways (hypoalgesia).

The compounds of the invention may also be useful in the treatment of inflammation, for example in the treatment of skin conditions (e.g. sunburn, bums, eczema, dermatitis, psoriasis); ophthalmic diseases such as glaucoma, retinitis, retinopathies, uveitis and of acute injury to the eye tissue (e.g. conjunctivitis); lung disorders (e.g. asthma, bronchitis, emphysema, allergic rhinitis, respiratory distress syndrome, pigeon fancier's disease, farmer's lung, COPD); gastrointestinal tract disorders (e.g. aphthous ulcer, Crohn's disease, atopic gastritis, gastritis varialoforme, ulcerative colitis, coeliac disease, regional ileitis, irritable bowel syndrome, inflammatory bowel disease, gastrointestinal reflux disease, diarrhoea, constipation); organ transplantation; other conditions with an inflammatory component such as vascular disease, migraine, periarteritis nodosa, thyroiditis, aplastic anaemia, Hodgkin's disease, sclerodoma, myaesthenia gravis, multiple sclerosis, sorcoidosis, nephrotic syndrome, Bechet's syndrome, polymyositis, gingivitis, myocardial ischemia, pyrexia, systemic lupus erythematosus, polymyositis, tendinitis, bursitis, and Sjogren's syndrome.

The compounds of the invention may also be useful in the treatment of immunological diseases such as autoimmune diseases, immunological deficiency diseases or organ transplantation. The compounds of the invention may also be effective in increasing the latency of HIV infection.

The compounds of the invention may also be useful in the treatment of diseases of excessive or unwanted platelet activation such as intermittent claudication, unstable angina, stroke, and acute coronary syndrome (e.g. occlusive vascular diseases).

The compounds of the invention may also be useful as a drug with diuretic action, or may be useful to treat overactive bladder syndrome.

The compounds of the invention may also be useful in the treatment of impotence or erectile dysfunction.

The compounds of the invention may also be useful in the treatment of bone disease characterised by abnormal bone metabolism or resorption such as osteoporosis (especially postmenopausal osteoporosis), hyper-calcemia, hyperparathyroidism, Paget's bone diseases, osteolysis, hypercalcemia of malignancy with or without bone metastases, rheumatoid arthritis, periodontitis, osteoarthritis, ostealgia, osteopenia, calculosis, lithiasis (especially urolithiasis), gout and ankylosing spondylitis, tendinitis and bursitis.

The compounds of the invention may also be useful in bone remodelling and/or promoting bone generation and/or promoting fracture healing.

The compounds of the invention may also be useful for attenuating the hemodynamic side effects of NSAIDs and COX-2 inhibitors.

The compounds of the invention may also be useful in the treatment of cardiovascular diseases such as hypertension or myocardial ischemia; functional or organic venous insufficiency; varicose therapy; haemorrhoids; and shock states associated with a marked drop in arterial pressure (e.g. septic shock).

The compounds of the invention may also be useful in the treatment of neurodegenerative diseases and neurodegeneration such as dementia, particularly degenerative dementia (including senile dementia, Alzheimer's disease, Pick's disease, Huntingdon's chorea, Parkinson's disease and Creutzfeldt-Jakob disease, ALS, motor neuron disease); vascular dementia (including multi-infarct dementia); as well as dementia associated with intracranial space occupying lesions; trauma; infections and related conditions (including HIV infection); metabolism; toxins; anoxia and vitamin deficiency; and mild cognitive impairment associated with ageing, particularly Age Associated Memory Impairment.

The compounds of the invention may also be useful in the treatment of neurological disorders and may be useful as neuroprotecting agents. The compounds of the invention may also be useful in the treatment of neurodegeneration following stroke, cardiac arrest, pulmonary bypass, traumatic brain injury, spinal cord injury or the like.

The compounds of the invention may also be useful in the treatment of complications of Type 1 diabetes (e.g. diabetic microangiopathy, diabetic retinopathy, diabetic nephropathy, macular degeneration, glaucoma), nephrotic syndrome, aplastic anaemia, uveitis, Kawasaki disease and sarcoidosis.

The compounds of the invention may also be useful in the treatment of kidney dysfunction (nephritis, particularly mesangial proliferative glomerulonephritis, nephritic syndrome), liver dysfunction (hepatitis, cirrhosis) and gastrointestinal dysfunction (diarrhoea).

It is to be understood that reference to treatment includes both treatment of established symptoms and prophylactic treatment.

According to a further embodiment of the invention, there is provided a compound of the invention or a salt thereof for use in human or veterinary medicine.

According to another embodiment of the invention, there is provided a compound of the invention or a salt thereof for use in the treatment of a condition which is mediated by the action, or loss of action, of PGE₂ at EP₄ receptors.

According to another embodiment of the invention, there is provided the use of a compound of the invention or a salt thereof for the manufacture of a medicament for the treatment of a condition which is mediated by the action of PGE₂ at EP₄ receptors.

According to another embodiment of the invention there is provided the use of a compound of the invention or a salt derivative thereof for the manufacture of a medicament for the treatment or prevention of a condition such as a pain, inflammatory, immunological, bone, neurodegenerative or renal disorder.

The compounds of the invention and their salts are conveniently administered in the form of pharmaceutical compositions. Such compositions may conveniently be presented for use in conventional manner in admixture with one or more physiologically acceptable carriers or excipients.

Thus, in another aspect of the invention, there is provided a pharmaceutical composition comprising a compound of the invention or a salt thereof adapted for use in human or veterinary medicine.

While it is possible for the compounds of the invention or a salt thereof to be administered as the raw chemical, it is preferable to present it as a pharmaceutical formulation. The formulations of the present invention comprise the compounds of the invention or a salt thereof together with one or more acceptable carriers or diluents therefor and optionally other therapeutic ingredients. The carrier(s) must be "acceptable" in the sense of being compatible with the other ingredients of the formulation and not deleterious to the recipient thereof.

The formulations include those suitable for oral, parenteral (including subcutaneous e.g. by injection or by depot tablet, intradermal, intrathecal, intramuscular e.g. by depot and intravenous), rectal and topical (including dermal, buccal and sublingual) administration although the most suitable route may depend upon for example the condition and disorder of the recipient. The formulations may conveniently be presented in unit dosage form and may be prepared by any of the methods well known in the art of pharmacy (see for example methods disclosed in 'Remingtom - The Science and Practice of Pharmacy', 21st Edition, Lippincott, Williams & Wilkins, USA, 2005 and references therein). All methods include the step of bringing into association the compound of the invention or a pharmaceutically acceptable acid addition salt thereof ("active ingredient") with the carrier which constitutes one or more accessory ingredients. In general the formulations are prepared by uniformly and intimately bringing into association the active ingredient with liquid carriers or finely divided solid carriers or both and then, if necessary, shaping the product into the desired formulation.

Formulations of the present invention suitable for oral administration may be presented as discrete units such as capsules, cachets or tablets (e.g. chewable tablets in particular for paediatric administration) each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein.

Formulations for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilised) condition requiring only the addition of a sterile liquid carrier, for example, water-for-injection, immediately prior to use. Extemporaneous injection solutions and suspensions may be prepared from sterile powders, granules and tablets of the kind previously described.

Formulations for rectal administration may be presented as a suppository with the usual carriers such as cocoa butter, hard fat or polyethylene glycol.

Formulations for topical administration in the mouth, for example buccally or sublingually, include lozenges comprising the active ingredient in a flavoured basis such as sucrose and acacia or tragacanth, and pastilles comprising the active ingredient in a basis such as gelatin and glycerin or sucrose and acacia.

The compounds of the invention may also be formulated as depot preparations. Such long acting formulations may be administered by implantation (for example subcutaneously or intramuscularly) or by intramuscular injection. Thus, for example, the compounds of the invention may be formulated with suitable polymeric or hydrophobic materials (for example as an emulsion in an acceptable oil) or ion exchange resins, or as sparingly soluble derivatives, for example, as a sparingly soluble salt.

In addition to the ingredients particularly mentioned above, the formulations may include other agents conventional in the art having regard to the type of formulation in question, for example those suitable for oral administration may include flavouring agents.

The EP₄ receptor compounds for use in the present invention may be used in combination with other therapeutic agents, for example COX-2 inhibitors, such as celecoxib, rofecoxib, valdecoxib or parecoxib; 5-lipoxygenase inhibitors; analgesics such as paracetamol; NSAID's, such as diclofenac, indomethacin, nabumetone, naproxen or ibuprofen; leukotriene receptor antagonists; DMARD's such as methotrexate; sodium channel blockers, such as lamotrigine; N-type calcium channel antagonists; NMDA receptor modulators, such as glycine receptor antagonists; gabapentin, pregabalin and related compounds; tricyclic antidepressants such as amitriptyline; neurone stabilising antiepileptic drugs; mono-aminergic uptake inhibitors such as venlafaxine; opioid analgesics; local anaesthetics; 5HT₁ agonists, such as triptans, for example sumatriptan, naratriptan, zolmitriptan, eletriptan, frovatriptan, almotriptan or rizatriptan; EP₁ receptor ligands; EP₂ receptor ligands; EP₃ receptor ligands; EP₁ antagonists; EP₂ antagonists and EP₃ antagonists; cannabanoid receptor agonists; VR1 antagonists. When the compounds are used in combination with other therapeutic agents, the compounds may be administered either sequentially or simultaneously by any convenient route.

The invention thus provides, in a further embodiment, a combination comprising a compound of the invention or a salt thereof together with a further therapeutic agent or agents. In one embodiment of the invention there is provided a combination comprising a compound of formula (I) or a salt thereof and paracetamol.

The combinations referred to above may conveniently be presented for use in the form of a pharmaceutical formulation and thus pharmaceutical formulations comprising a combination as defined above together with a pharmaceutically acceptable carrier or excipient comprise a further aspect of the invention. The individual components of such combinations may be administered either sequentially or simultaneously in separate or combined pharmaceutical formulations.

When a compound of the invention or a salt thereof is used in combination with a second therapeutic agent active against the same disease, the dose of each compound may differ from that when the compound is used alone. Appropriate doses will be readily appreciated by those skilled in the art.

A proposed daily dosage of a compound of the invention, or a pharmaceutically acceptable salt thereof, for the treatment of man is from 0.001 to 30 mg/kg body weight per day and more particularly 0.1 to 3 mg/kg body weight per day, calculated as the free acid, which may be administered as a single or divided dose, for example one to four times per day. The dose range for adult human beings is generally from 0.1 to 1000 mg/day, such as from 10 to 800 mg/day, preferably 10 to 200 mg/day, calculated as the free acid.

In one embodiment, when compounds of Formula (I) or salts thereof, are administered in combination with paracetamol, a suitable daily dosage of paracetamol is up to 4000 mg per day. Suitable unit doses include 200, 400, 500 and 1000 mg, one, two, three or four times per day.

The precise amount of the compounds of the invention administered to a host, particularly a human patient, will be the responsibility of the attendant physician. However, the dose employed will depend on a number of factors including the age and sex of the patient, the precise condition being treated and its severity, the route of administration, and any possible combination therapy that may be being undertaken.

The compounds of the invention may be prepared according to Schemes 1, 2 and 3 in which they are denoted generally as "Formula (I)". 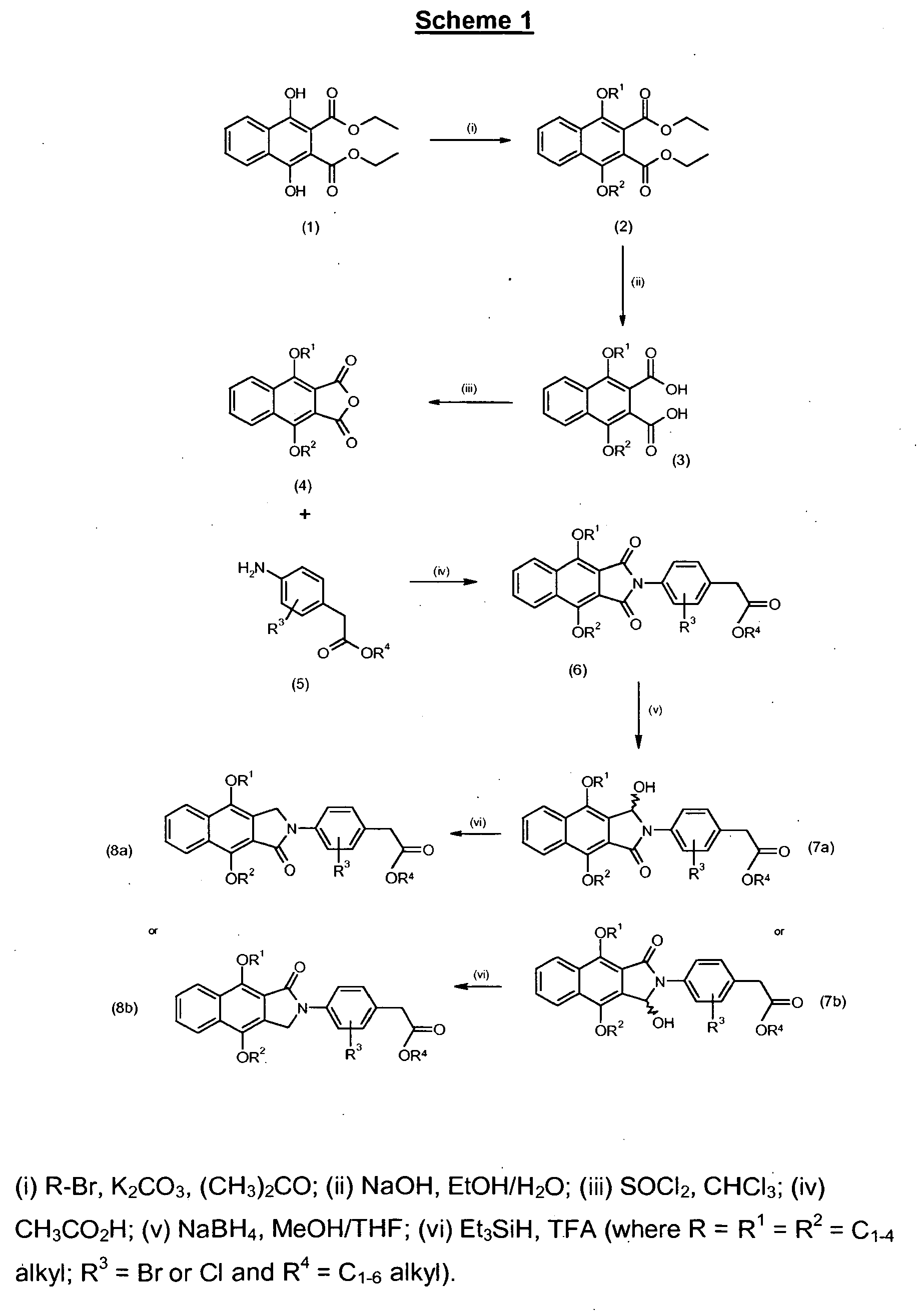 Compound (1) may be prepared in accordance with the method disclosed in International Patent Application, Publication Number WO02/064564.

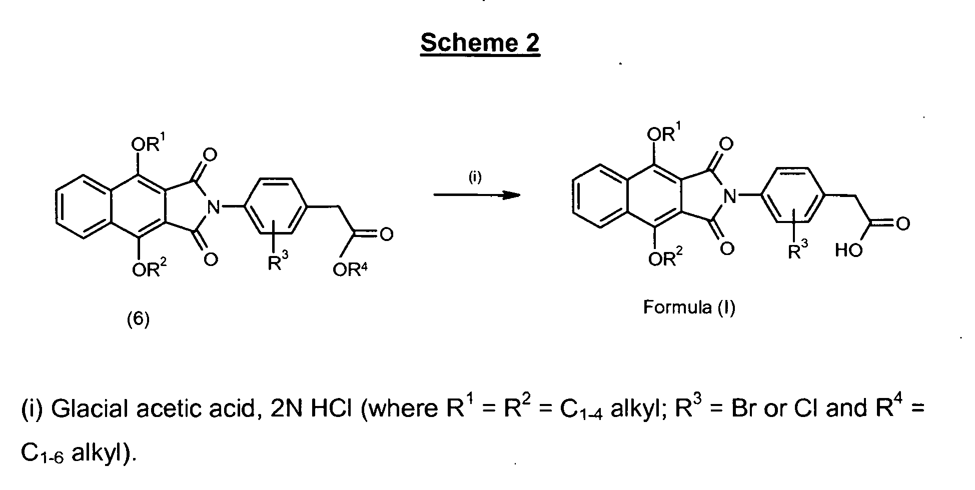

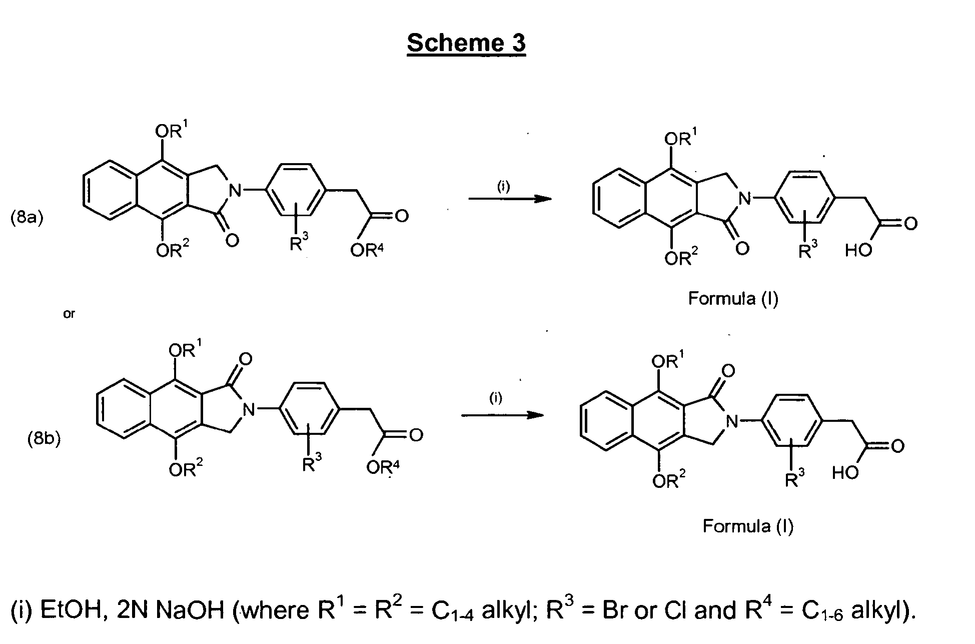

Compounds of formula (5) may be prepared according to Scheme 4:

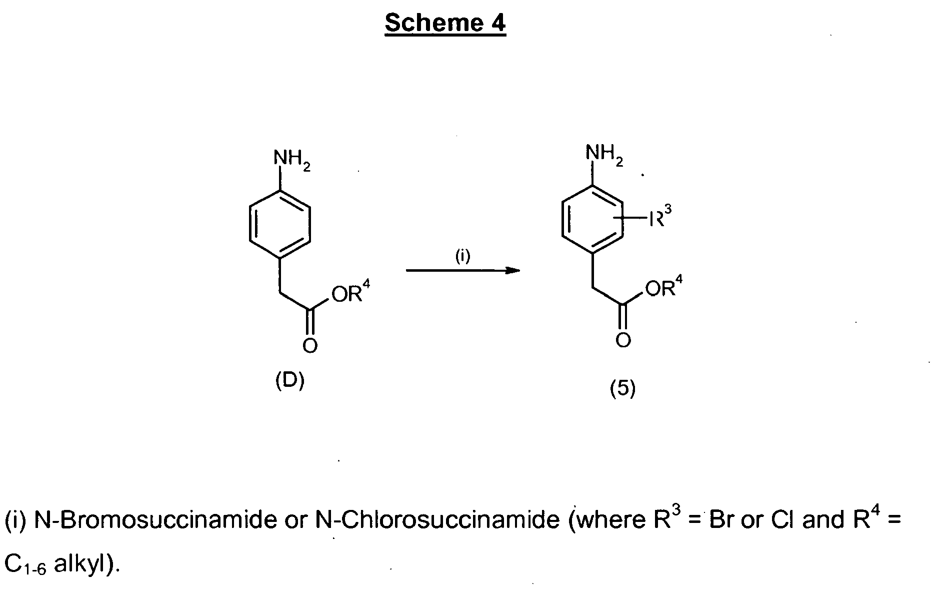

Compounds of formula (D) are commercially available or may be prepared in accordance with methods known in the art (for example, ethyl 4-aminophenyl acetate may be purchased from Avocado Research).

The following Descriptions and Examples illustrate the preparation of compounds of formula (I). Descriptions refer to intermediate compounds and Examples refer to compounds of formula (I). The starting material for the preparation of intermediates may not necessarily have been prepared from the batch referred to. The intermediates for the preparation of the examples may not necessarily have been prepared from the batch referred to.

### Abbreviations

- DMAP: 4-(Dimethylamino)pyridine
- EtOH: Ethanol
- IPA: Isopropyl alcohol
- MeOH: Methanol
- NaOH: Sodium hydroxide
- TFA: Trifluoroacetic acid
- THF: Tetrahydrofuran

### Analytical procedures

### LC/MS

### Column

Waters Atlantis (4.6mm x 50mm). Stationary phase particle size; 3µm.

### Solvents

A: Aqueous solvent = Water + 0.05% Formic Acid
B: Organic solvent = Acetonitrile + 0.05% Formic Acid

### Method

| Time / min | %B |
|---|---|
| 0 | 3 |
| 0.1 | 3 |
| 4 | 97 |
| 4.8 | 97 |
| 4.9 | 3 |
| 5.0 | 3 |

- Flow rate, 3ml/mins.
- Injection volume, 5µl.
- Column temperature, 30°C.
- UV detection range, 220 to 330nm.

All retention times are measured in minutes.

### Purification Techniques

Purification of the Examples may be carried out by conventional methods such as chromatography and/or recrystallisation using suitable solvents. Chromatographic methods include column chromatography, flash chromatography, HPLC (high performance liquid chromatography), SFC (supercritical fluid chromatography), and MDAP (mass directed autopreparation). The term "Biotage" when used herein refers to commercially available prepacked silica gel cartridges.

### Description 1

### Diethyl 1,4-bis(propyloxy)-2,3-naphthalenedicarboxylate

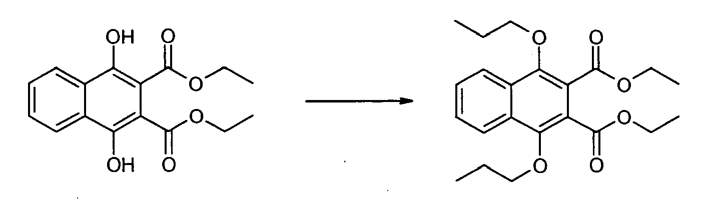

1-Bromopropane (13.1ml, 144.4mmol) was added to a stirring solution of diethyl 1,4-dihydroxy-2,3-naphthalenedicarboxylate (11g, 36.1mmol) [WO 02/064564] and potassium carbonate (24.9g, 180.5mmol) in acetone (180ml). Refluxed overnight under an atmosphere of argon. The resulting mixture was cooled, filtered and the solvent evaporated from the filtrate. The residue was taken up in toluene and washed with 5% potassium hydroxide solution, brine and dried over magnesium sulphate. Purified by chromatography on silica gel eluting with ethyl acetate/hexane (1:9) to give the title compound as yellow oil (11.45g, 29.5mmol). LC/MS: Rt=3.87, [MH]⁺ 389.

The following compounds were prepared in a similar manner to diethyl 1,4-bis(propyloxy)-2,3-naphthalenedicarboxylate using the appropriate starting materials.

| **Description** | | **Name** | **LC/MS** |
|---|---|---|---|
| Description 2 | 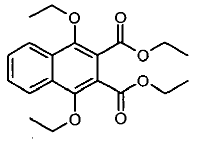 | *Diethyl 1,4-bis(ethyloxy)-*2,3-*naphthalenedicarboxylate* | Rt=3.49 [MH]⁺ 361 |
| Descritpion 3 | 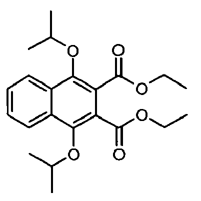 | *Diethyl 1,4-bis(1-methylethoxy)-2,3-naphthalenedicarboxylate* | Rt=3.63 [MH]⁺ 389 |

### Description 4

### 1,4-Bis(propyloxy)-2,3-naphthalenedicarboxylic acid

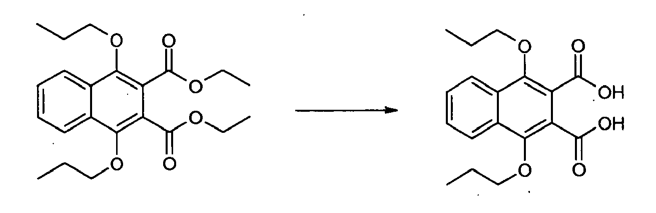

A mixture of diethyl 1,4-bis(propyloxy)-2,3-naphthalenedicarboxylate (11.45g, 29.5mmol), ethanol (70ml), sodium hydroxide (3.54g, 88.5mmol) and water (15ml) was refluxed for 4 hours. The reaction mixture was cooled and evaporated to a third of the volume. This was acidified with hydrochloric acid (2N) and extracted with ethyl acetate (3x 100ml). Combined organics washed with water, brine and dried over magnesium sulphate. Solvent was evaporated to give the title compound as a yellow solid (8.91g, 26.8mmol). LC/MS: Rt=2.74, [MH]⁺ 333.

The following compounds were prepared in a similar manner to 1,4-bis(propyloxy)-2,3-naphthalenedicarboxylic acid using the appropriate starting materials.

| **Description** | | **Name** | **LC/MS** |
|---|---|---|---|
| Description 5 | 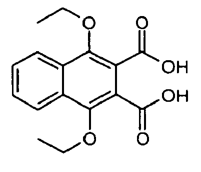 | *1,4-Bis(ethyloxy)-2,3-naphthalenedicarboxylic acid* | Rt=2.38 [MH]⁺ 305 |
| Description 6 | 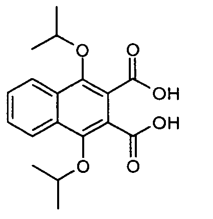 | *1,4-Bis(1-methylethoxy)-*2,3-*naphthalenedicarboxylic acid* | Rt=2.50 [MH]⁻ 331 |

### Description 7

### 4,9-Bis(propyloxy)naphtho[2,3-c]furan-1,3-dione

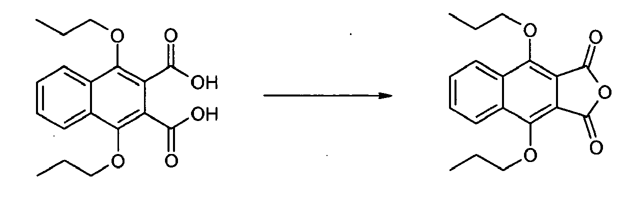

Thionyl chloride (20.5ml, 281.4mmol) was added dropwise to a solution of 1,4-bis(propyloxy)-2,3-naphthalenedicarboxylic acid (8.91g, 26.8mmol) in chloroform (80ml) and heated at 65°C for 2.5 hours. The reaction mixture was cooled and solvent evaporated to yellow solid. Azeotroped with chloroform to give the title compound as a beige solid (8.74g, 27.8mmol). LC/MS: Rt=3.77, [MH]⁺ 315.

The following compounds were prepared in a similar manner to 4,9-bis(propyloxy)naphtho[2,3-c]furan-1,3-dione using the appropriate starting materials.

| **Description** | | **Name** | **LC/MS** |
|---|---|---|---|
| Description 8 | 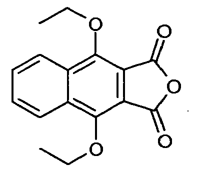 | *4,9-Bis(ethyloxy)naphtho[2,3-c]furan-1,3-dione* | Rt=3.48 [MH]⁺ 287 |
| Description 9 | 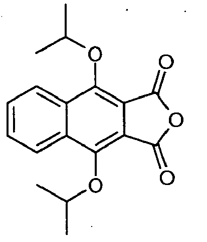 | *4,9 Bis(1-methylethoxy)naphtho[2,3-c]furan-1,3-dione* | Rt=3.6 [MH]⁺ 315 |

### Description 10

### Ethyl (4-amino-3-bromophenyl)acetate

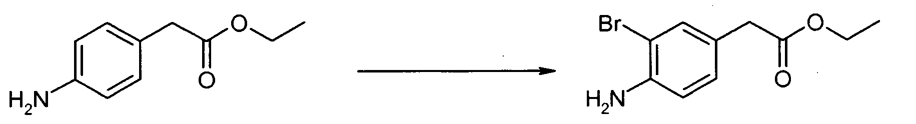

Ethyl-4-aminophenylacetate (5g, 27.9mmol) was dissolved in chloroform (100ml) and treated with N-bromosuccinimde (4.97g, 27.9mmol) and stirred for one hour at room temperature under argon. Reaction mixture was washed with water, brine and dried over magnesium sulphate. Evaporated to a brown oil which was purified by chromatography on silica gel eluting with ethyl acetate/hexane (1:3) to give the title compound as a yellow oil (6.03g, 23.4mmol). LC/MS: Rt=2.55, [MH]⁺ 260.

The following compound was prepared in a similar manner to ethyl (4-amino-3-bromophenyl) acetate, using the appropriate starting materials.

| **Description** | | **Name** | **LC/MS** |
|---|---|---|---|
| Description 11 | 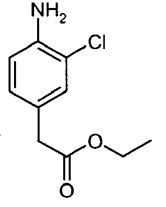 | *Ethyl (4-amino-3-chlorophenyl)acetate* | Rt=2.53 [MH]⁺ 214/216 |

### Description 12

### Ethyl {4-[4,9-bis(ethyloxy)-1,3-dioxo-1,3-dihydro-2H-benzo[f]isoindol-2-yl]-3-chlorophenyl}acetate

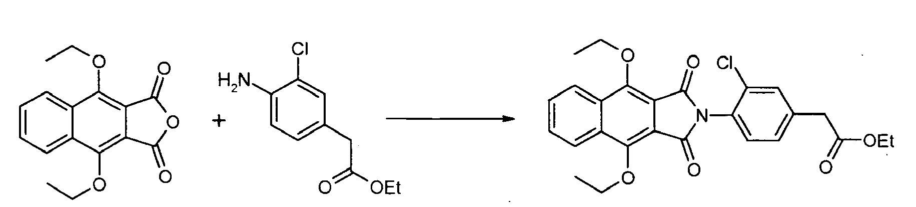

A stirring suspension of 4,9-bis(ethyloxy)naphtho[2,3-c]furan-1,3-dione (0.200g, 0.7mmol) in acetic acid (2ml) was treated with ethyl (4-amino-3-chlorophenyl)acetate (0.284g, 1.33mmol) and DMAP (0.024g, 0.21 mmol).This was refluxed overnight with molecular sieves under an argon atmosphere. The reaction mixture was cooled and the molecular sieves removed by filtration through celite, washing with acetic acid. Water was added with stirred until a precipitate formed. The solid was collected by filtration and washed with water. This was recrystallised from boiling IPA to give the title compound (0.102g, 0.21mmol). LC/MS: Rt=3.84, [MH]⁺ 482.

The following compounds were prepared in a similar manner to ethyl {4-[4,9-bis(ethyloxy)-1,3-dioxo-1,3-dihydro-2H-benzo[f]isoindol-2-yl]-3-chlorophenyl*}acetate* using the appropriate starting materials.

| **Description** | | **Name** | **LC/MS** |
|---|---|---|---|
| Description 13 | 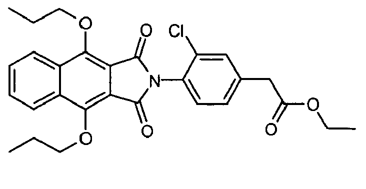 | *Ethyl [3-chloro-4-[1,3-dioxo-4,9-bis(propyloxy)-1, 3-dihydro-2H-benzo[f]isoindol-2-yl]phenyl}acetate* | Rt=4.19 [MH]⁺ 510 |
| Description 14 | 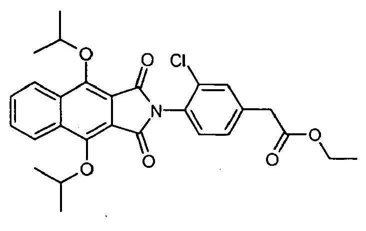 | *Ethyl (4-{4,9-bis(1-methylethoxy)-1,3-dioxo-1,3-dihydro-2H-benzo[f]isoindol-2-yl}-3-chlorophenyl)aceta te* | Rt=4.00 [MH]⁺ 510 |
| Description 15 | 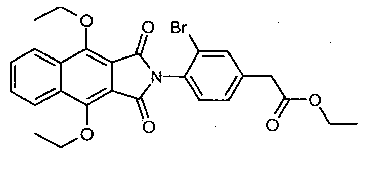 | *Ethyl {4-[4,9-bis(ethyloxy)-1,3-dioxo-1,3-dihydro-2H-benzo[f]isoindol-2-yl]-3-bromophenyl}acet ate* | Rt=3.77 [MH]⁺ 528 |
| Description 16 | 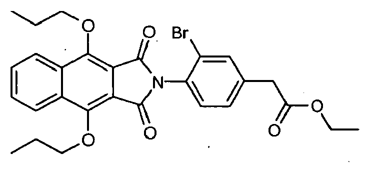 | *Ethyl {3-bromo-4-[1,3-dioxo-4,9-bis(propyloxy)-,1,3-dihydro-2H-benzo[f]isoindol-2-yl]phenyl}acetate* | Rt=4.13 [MH]⁺ 556 |
| Description 17 | 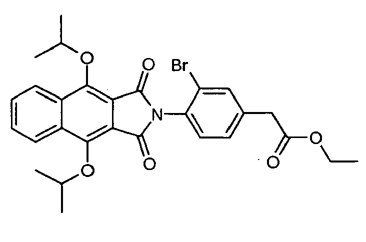 | *Ethyl (4-{4,9-bis(1-methylethoxy)-1,3-dioxo-1,3-dihydro-2H-benzo[f]isoindol-2-yl}-3-bromophenyl)acet ate* | Rt=4.06 [MH]⁺ 556 |

### Example 1

### {4-[4,9-Bis(ethyloxy)-1,3-dioxo-1,3-dihydro-2H-benzo[f]isoindol-2-yl]-3-chlorophenyl}acetic acid

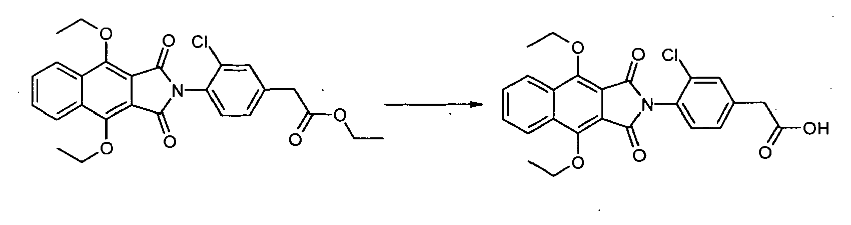

Ethyl {4-[4,9-bis(ethyloxy)-1,3-dioxo-1,3-dihydro-2*H*-benzo[*f*]isoindol-2-yl]-3-chlorophenyl}acetate (0.120g, 0.25mmol) was suspended in glacial acetic acid (4ml) and 2N hydrochloric acid (4ml), and heated until complete, (100°C, 2-5 hours). Cooled and water was added. The white precipitate which formed was filtered, washed with water, collected and dried in a vacuum oven overnight to give the title compound as a white solid, (0.084g, 0.19mmol). LC/MS: Rt=3.39, (MH]⁺ 454.

The following compounds were prepared in a similar manner to {4-[4,9-bis(ethyloxy)-1,3-dioxo-1,3-dihydro-2*H*-benzo[*f*]isoindol-2-yl]-3-chlorophenyl}acetic acid using the appropriate staring materials.

| **Example** | | **Name** | **LC/MS** |
|---|---|---|---|
| **Example 2** | 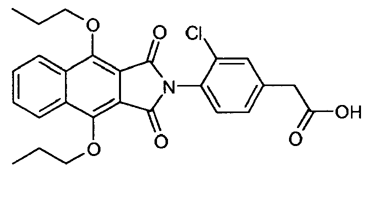 | *{3-Chloro-4-[1,3-dioxo-4,9-bis(propyloxy)-1,3-dihydro-2H-benzo[f]isoindol-2-yl]phenyl}acetic acid* | Rt=3.75 [MH]⁺ 482 |
| **Example 3** | 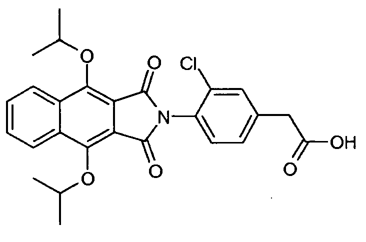 | *(4-{4,9-Bis(1-methylethoxy)-1,3-dioxo-1,3-dihydro-2H-benzo[f]isoindol-2-yl}-3-chlorophenyl)acetic acid* | Rt=3.56 [MH]⁺ 482 |
| **Example 4** | 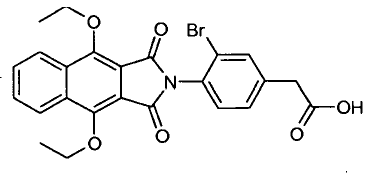 | *{4-[4,9-Bis(ethyloxy)-1,3-dioxo-1,3-dihydro-2H-benzo[f]isoindol-2-yl]-*3-*bromophenyl}acetic acid* | Rt=3.44 [MH]⁺ 500 |
| **Example 5** | 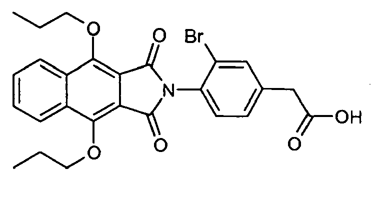 | {*3-Bromo-4-[1,3-dioxo-4,9-bis(propyloxy)-1,3-dihydro-2H-benzo[f]isoindol-2-yl]phenyl}acetic acid* | Rt=3.75 [MH]⁺ 528 |
| **Example 6** | 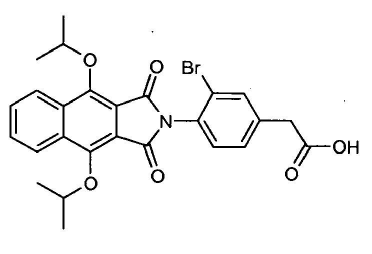 | *(4-{4,9-Bis(1-methylethoxy)-1,3-dioxo-1,3-dihydro-2H-benzo[f]isoindol-2-yl}-3-bromophenyl)acetic acid* | Rt= 3.60 [MH]⁺ 528 |

### Description 18

### Ethyl (3-chloro-4-{1-hydroxy-4,9-bis(1-methylethoxy)-3-oxo-1,3-dihydro-2H-benzo[f]isoindol-2-yl}phenyl)acetate

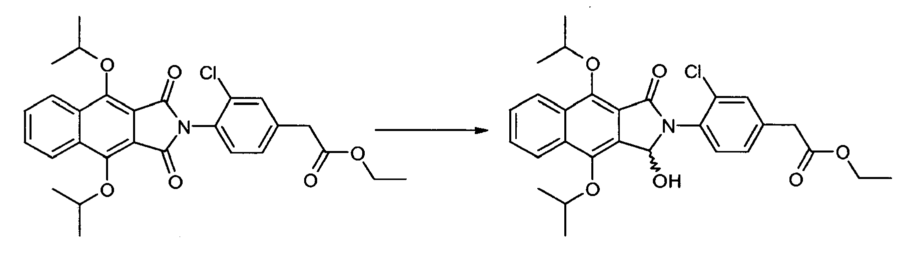

Ethyl (4-{4,9-bis(1-methylethoxy)-1,3-dioxo-1,3-dihydro-2*H*-benzo[*f*]isoindol-2-yl}-3-chlorophenyl)acetate (0.500g, 0.98mmol) was suspended in methanol (1ml), ethanol (2ml) and tetrahydrofuran (4ml) was added to aid solubility. Cooled to 0°C in an ice bath and treated with sodium borohydride (0.111g, 2.94mmol) portionwise over 2 minutes. Stirred under argon for 2 hours at 0°C. Solvent evaporated and the residue was partitioned between ethyl acetate and 1 M ammonium chloride solution. The aqueous layer was extracted with ethyl acetate (x2). Combined organics washed with brine and dried over magnesium sulphate. Organics evaporated to give the title compound (0.499g, 0.97mmol). LC/MS: Rt=3.71, [MH]⁺ 512.

The following compounds were prepared in a similar manner to ethyl (3-chloro-4-{1-hydroxy-4,9-bis(1-methylethoxy)-3-oxo-1,3-dihydro-2*H*-benzo[*f*]isoindol-2-yl}phenyl)acetate using the appropriate starting materials.

| **Description** | | **Name** | **LC/MS** |
|---|---|---|---|
| Description 19 | 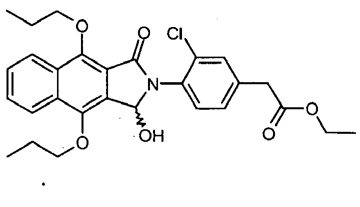 | *Ethyl {3-chloro-4-[1-hydroxy-3-oxo-* 4,9-*bis(propyloxy)-1,3-dihydro-2H-benzo[f]isoindol-*2-*yl]phenyl}acetate* | Rt=3.88 [MH]⁺ 512 |
| Description 20 | 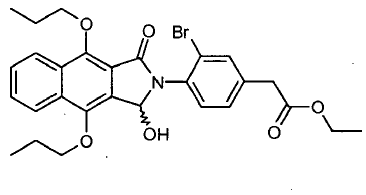 | *Ethyl {3-promo-4-[1-hydroxy-3-oxo-* 4,9-*bis(propyloxy)-1,3-dihydro-2H-benzo[f]isoindol-*2-*yl]phenyl}acetate* | Rt=3.91 [MH]⁺ 558 |
| Description 21 | 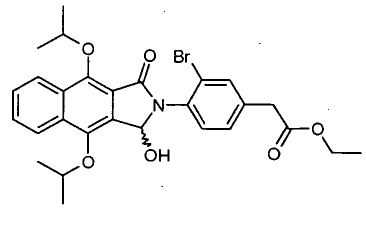 | *Ethyl (3-bromo-4-{1-hydroxy-4,9-bis(1-methylethoxy)-3-oxo-1,3-dihydro-2H-benzo[f]isoindol-*2-*yl}phenyl)acetate* | Rt=3.75 [MH]⁺ 558 |

### Description 22

### Ethyl (4-{4,9-bis(1-methylethoxy)-1-oxo-1,3-dihydro-2H-benzo[f]isoindol-2-yl}-3-chlorophenyl)acetate

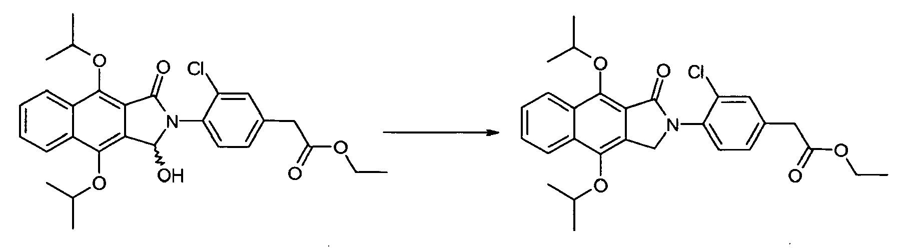

Ethyl (3-chloro-4-{1-hydroxy-4,9-bis(1-methylethoxy)-3-oxo-1,3-dihydro-2H-benzo[*f*]isoindol-2-yl}phenyl)acetate (0.499g, 0.97mmol) was dissolved in trifluoroacetic acid (2ml) and cooled in an ice bath to 0°C. Treated with triethylsilane (0.23ml, 1.46mmol) and stirred at 0°C for 1 hour. Volatiles evaporated and purified directly by chromatography on silica gel to give the title compound (0.180g, 0.36 mmol). LC/MS: Rt=3.93, [MH]⁺ 496.

The following compounds were prepared in a similar manner to ethyl (4-{4,9-bis(1-methylethoxy)-1-oxo-1,3-dihydro-2*H*-benzo[*f*]isoindol-2-yl}-3-chlorophenyl)acetate using the appropriate starting materials.

| **Description** | | **Name** | **LC/MS** |
|---|---|---|---|
| Description 23 | 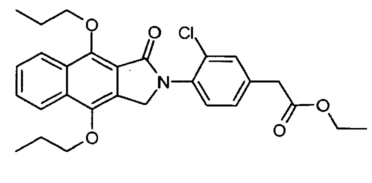 | *Ethyl {3-chloro-4-[1-oxo-4,9-bis(propyloxy)-1,3-dihydro-2H-benzo[f]isoindol-*2-*yl]phenyl}acetate* | Rt=4.21 [MH]⁺ 496 |
| Description 24 | 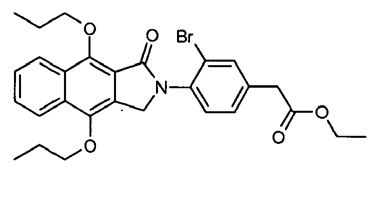 | *Ethyl {3-bromo-4-[1-oxo-4,9-bis(propyloxy)-1,3-dihydro-2H-benzo[f]isoindol-* 2-*yl]phenyl}acetate* | Rt=4.23 [MH]⁺ 542 |
| Description 25 | 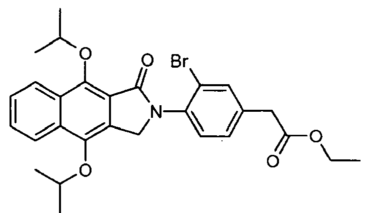 | *Ethyl (4-{4,9-bis(1-methylethoxy)-1-oxo-1,3-dihydro-2H-benzo[f]isoindol-2-yl}-3-bromophenyl)acetate* | Rt=4.08 [MH]⁺ 542 |

### Example 7

### (4-{4,9-Bis(1-methylethoxy)-1-oxo-1,3-dihydro-2H-benzo[f]isoindol-2-yl}-3-chlorophenyl)acetic acid

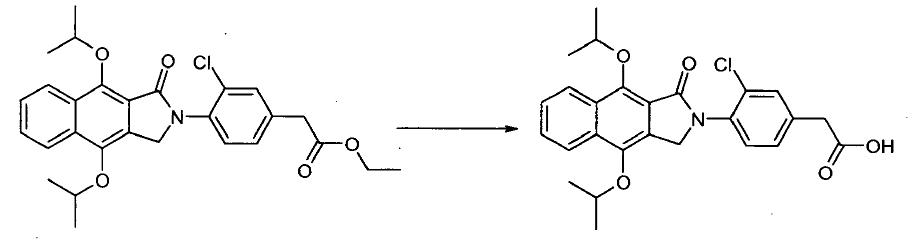

Ethyl (4-{4,9-bis(1-methylethoxy)-1-oxo-1,3-dihydro-2*H*-benzo[*f*]isoindol-2-yl}-3-chlorophenyl)acetate (0.160g, 0.32mmol) was dissolved in ethanol (3ml) and treated with 2N sodium hydroxide (5ml). Heated to reflux for one hour then cooled, and the solvent evaporated. The residue was acidified with 2N hydrochloric acid to pH1. This was extracted with ethyl acetate (x2), washed with brine and dried over magnesium sulphate. The solvent was evaporated and then the residue triturated with hexane to give the title compound as a white solid (0.125g, 0.26mmol). LC/MS: Rt=3.53, [MH]⁺ 468.

The following compounds were prepared in a similar manner to (4-{4,9-bis(1-methylethoxy)-1-oxo-1,3-dihydro-2*H*-benzo[*f*]isoindol-2-yl}-3-chlorophenyl)acetic acid using the appropriate starting materials.

| **Example** | | **Name** | **LC/MS** |
|---|---|---|---|
| **Example 8** | 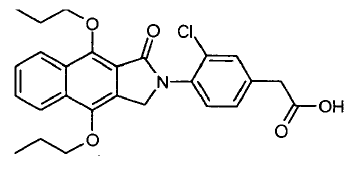 | *{3-Chloro-4-[1-oxo-4,9-bis(propyloxy)-1,3-dihydro-2H-benzo[f]soindol-2-yl]phenyl}acetic acid* | Rt=3.72 [MH]⁺ 468 |
| **Example 9** | 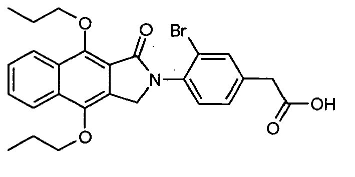 | *{3-Bromo-4-[1-oxo-4,9-bis(propyloxy)-1,3-dihydro-2H-benzo[f]isoindol-2-yl]phenyl}acetic acid* | Rt=3.74 [MH]⁺ 514 |
| **Example 10** | 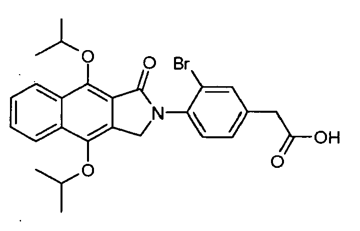 | (4-{4,9-Bis(1-methylethoxy)-1-oxo-1,3-dihydro-2*H*-benzo[*f*]isoindol-2-yl}-3-bromophenyl)acetic acid | Rt=3.55 [MH]⁺ 514 |

### Biological data

Studies were performed using HEK-293(T) cells expressing the recombinant human prostanoid EP₄ receptor (HEK-EP₄ cells). Cells were grown as a monolayer culture in DMEM-F12/F12 containing glutamax II (Gibco) and supplemented with 10% foetal bovine serum and 0.4mg.ml-1 G418. HEK-EP₄ cells were pre-treated 24hr and 30mins prior to the experiment with 10µM indomethacin and harvested using Versene containing 10µM indomethacin. The cells were resuspended in assay buffer (DMEM:F12, 10µM indomethacin and 200µM IBMX) at 1×10⁶ cells per ml and incubated for 20min at 37°C. Thereafter, 50µl of cells were added to 50µl agonist (compound of Formula (I)) and incubated at 37°C for 4 minutes before stopping reactions with 100µl of 1% triton X-100. cAMP levels in the cell lysates were determined using a competition binding assay. In this assay the ability of cell lysates to inhibit 3H-cAMP (Amersham) binding to the binding subunit of protein kinase A was measured and cAMP levels were calculated from a standard curve. The data for each compound were expressed as a % of the response to a 10nM maximal concentration of the standard agonist PGE2. For each compound the maximal response and concentration of compound causing 50% of its maximal response were calculated. Intrinsic activity is expressed relative to the maximal response to PGE2. Unless stated, reagents were purchased commercially from Sigma.

The examples of the present invention were tested in the above-mentioned assay and exhibited pEC₅₀ values of 6.6 or higher and intrinsic activities of 60% or higher.

## Claims

1. A compound selected from the group consisting of:
{4-[4,9-Bis(ethyloxy)-1,3-dioxo-1,3-dihydro-2*H*-benzo[*f*]isoindol-2-yl]-3-chlorophenyl}acetic acid;
{3-Chloro-4-[1,3-dioxo-4,9-bis(propyloxy)-1,3-dihydro-2*H*-benzo[*f*]isoindol-2-yl]phenyl}acetic acid;
(4-{4,9-Bis(1-methylethoxy)-1,3-dioxo-1,3-dihydro-2*H*-benzo[*f*]isoindol-2-yl}-3-chlorophenyl)acetic acid;
{4-[4,9-Bis(ethyloxy)-1,3-dioxo-1,3-dihydro-2*H*-benzo[*f*]isoindol-2-yl]-3-bromophenyl}acetic acid;
{3-Bromo-4-[1,3-dioxo-4,9-bis(propyloxy)-1,3-dihydro-2*H*-benzo[*f*]isoindol-2-yl]phenyl}acetic acid;
(4-{4,9-Bis(1-methylethoxy)-1,3-dioxo-1,3-dihydro-2*H*-benzo[*f*]isoindol-2-yl}-3-bromophenyl)acetic acid;
(4-{4,9-Bis(1-methylethoxy)-1-oxo-1,3-dihydro-2*H*-benzo[*f*]isoindol-2-yl}-3-chlorophenyl)acetic acid;
{3-Chloro-4-[1-oxo-4,9-bis(propyloxy)-1,3-dihydro-2*H*-benzo[*f*]isoindol-2-yl]phenyl}acetic acid;
{3-Bromo-4-[1-oxo-4,9-bis(propyloxy)-1,3-dihydro-2*H*-benzo[*f*]isoindol-2-yl]phenyl}acetic acid;
(4-{4,9-Bis(1-methylethoxy)-1-oxo-1,3-dihydro-2*H*-benzo[*f*]isoindol-2-yl}-3-bromophenyl)acetic acid; and salts thereof.

2. A compound according to claim 1 or a pharmaceutically acceptable salt thereof for use in human or veterinary medicine.

3. A compound according to claim 1 or a pharmaceutically acceptable salt thereof, for use in the treatment of a condition which is mediated by the action, or loss of action, of PGE₂ at EP₄ receptors.

4. Use of a compound according to claim 1 or a pharmaceutically acceptable salt thereof, for the manufacture of a medicament for the treatment of a condition which is mediated by the action of PGE₂ at EP₄ receptors.

5. Use according to claim 4, wherein the condition is selected from the group consisting of a pain, inflammatory, immunological, bone, neurodegenerative or renal disorder.

6. A pharmaceutical composition comprising a compound according to claim 1 or a pharmaceutically acceptable salt thereof and one or more acceptable carriers or diluents therefor.

7. A pharmaceutical composition according to claim 6, comprising one or more additional therapeutic agents.

## Patentansprüche

1. Eine Verbindung, ausgewählt aus der Gruppe bestehend aus:
{4-[4,9-Bis(ethyloxy)-1,3-dioxo-1,3-dihydro-2H-benzo[f]isoindol-2-yl]-3-chlorphenyl}-essigsäure;
{3-Chlor-4-[1,3-dioxo-4,9-bis(propyloxy)-1,3-dihydro-2H-benzo[f]isoindol-2-yl]-phenyl}essigsäure;
(4-{4,9-Bis(1-methylethoxy)-1,3-dioxo-1,3-dihydro-2H-benzo[f]isoindol-2-yl}-3-chlorphenyl)essigsäure;
{4-[4,9-Bis(ethyloxy)-1,3-dioxo-1,3-dihydro-2H-benzo[f]isoindol-2-yl]-3-bromphenyl}essigsäure;
{3-Brom-4-[1,3-dioxo-4,9-bis(propyloxy)-1,3-dihydro-2H-benzo[f]isoindol-2-yl]-phenyl}essigsäure;
(4-{4,9-Bis(1-methylethoxy)-1,3-dioxo-1,3-dihydro-2H-benzo[f]isoindol-2-yl}-3-bromphenyl)essigsäure;
(4-{4,9-Bis(1-methylethoxy)-l-oxo-1,3-dihydro-2H-benzo[f]isoindol-2-yl}-3-chlorphenyl)essigsäure;
{3-Chlor-4-[1-oxo-4,9-bis(propyloxy)-1,3-dihydro-2H-benzo[f]isoindol-2-yl]phenyl}-essigsäure;
{3-Brom-4-[1-oxo-4,9-bis(propyloxy)-1,3-dihydro-2H-benzo[f]isoindol-2-yl]phenyl}-essigsäure;
(4-{4,9-Bis(1-methylethoxy)-1-oxo-1,3-dihydro-2H-benzo[f]isoindol-2-yl}-3-bromphenyl)essigsäure; und Salze davon.

2. Eine Verbindung gemäß Anspruch 1 oder ein pharmazeutisch verträgliches Salz davon zur Verwendung in der Human- oder Tiermedizin.

3. Eine Verbindung gemäß Anspruch 1 oder ein pharmazeutisch verträgliches Salz davon zur Verwendung bei der Behandlung eines Zustands, welcher durch die Wirkung oder den Verlust der Wirkung von PGE₂ an EP₄-Rezeptoren herbeigeführt wird.

4. Verwendung einer Verbindung gemäß Anspruch 1 oder eines pharmazeutisch verträglichen Salzes davon bei der Herstellung eines Medikaments zur Behandlung eines Zustandes, welcher durch die Wirkung von PGE₂ an EP₄ Rezeptoren herbeigeführt wird.

5. Verwendung gemäß Anspruch 4, wobei der Zustand ausgewählt ist aus der Gruppe bestehend aus Schmerz, Entzündung, Immunstörung, einer Störung der Knochen, neurodegenerativer Störung oder einer Störung der Nieren.

6. Ein Arzneimittel, umfassend eine Verbindung gemäß Anspruch 1 oder ein pharmazeutisch verträgliches Salz davon und einen oder mehrere verträgliche Träger oder Verdünnungsmittel dafür.

7. Ein Arzneimittel gemäß Anspruch 6, umfassend einen oder mehrere zusätzliche Wirkstoffe.

## Revendications

1. Composé choisi dans le groupe consistant en :
l'acide {4-[4,9-bis(éthyloxy)-1,3-dioxo-1,3-dihydro-2*H-*benzo[*f*]iso-indole-2-yl]-3-chlorophényl}acétique ;
l'acide {3-chloro-4-[1,3-dioxo-4,9-bis(propyloxy)-1,3-dihydro-2*H*-benzo[*f*]iso-indole-2-yl]phényl}acétique ;
l'acide (4-{4,9-bis(1-méthyléthoxy)-1,3-dioxo-1,3-dihydro-2H-benzo[*f*]iso-indole-2-yl}-3-chlorophényl)acétique ;
l'acide {4-[4,9-bis(éthyloxy)-1,3-dioxo-1,3-dihydro-2*H-*benzo[*f*]iso-indole-2-yl]-3-bromophényl}acétique ;
l'acide {3-bromo-4-[1,3-dioxo-4,9-bis(propyloxy)-1,3-dihydro-2*H*-benzo[*f*]iso-indole-2-yl]phényl}acétique ;
l'acide (4-{4,9-bis(1-méthyléthoxy)-1,3-dioxo-1,3-dihydro-2*H*-benzo[*f*]iso-indole-2-yl}-3-bromophényl)acétique ;
l'acide (4-{4,9-bis(1-méthyléthoxy)-1-oxo-1,3-dihydro-2*H*-benzo[*f*]iso-indole-2-yl}-3-chlorophényl)acétique ;
l'acide {3-chloro-4-[1-oxo-4,9-bis(propyloxy)-1,3-dihydro-2*H*-benzo[*f*]iso-indole-2-yl]phényl}acétique ;
l'acide {3-bromo-4-[1-oxo-4,9-bis(propyloxy)-1,3-dihydro-2*H*-benzo[*f*]iso-indole-2-yl]phényl}acétique ;
l'acide (4-{4,9-bis(1-méthyléthoxy)-1-oxo-1,3-dihydro-2*H*-benzo[*f*]iso-indole-2-yl}-3-bromophényl)acétique ;
et ses sels.

2. Composé suivant la revendication 1 ou un de ses sels pharmaceutiquement acceptables, destiné à être utilisé en médecine humaine ou vétérinaire.

3. Composé suivant la revendication 1 ou un de ses sels pharmaceutiquement acceptables, destiné à être utilisé dans le traitement d'une affection qui est soumise à une médiation par l'action, ou la perte d'action, de la PGE₂ au niveau des récepteurs EP₄.

4. Utilisation d'un composé suivant la revendication 1 ou d'un de ses sels pharmaceutiquement acceptables, pour la production d'un médicament destiné au traitement d'une affection qui est soumise à la médiation par l'action de la PGE₂ au niveau des récepteurs EP₄.

5. Utilisation suivant la revendication 4, dans laquelle l'affection est choisie dans le groupe consistant en un trouble douloureux, inflammatoire, immunologique, osseux, neurodégénératif et rénal.

6. Composition pharmaceutique comprenant un composé suivant la revendication 1 ou un de ses sels pharmaceutiquement acceptables et un ou plusieurs supports ou diluants acceptables à cette fin.

7. Composition pharmaceutique suivant la revendication 6, comprenant un ou plusieurs agents thérapeutiques supplémentaires.
